# EUROPÄISCHE PATENTSCHRIFT

(11) **EP 2 374 000 B1**
(45) Veröffentlichungstag und Bekanntmachung des Hinweises auf die Patenterteilung: **04.11.2015**
(21) Anmeldenummer: 10709950.9
(22) Anmeldetag: 08.01.2010
(51) Int. Cl.: G01N 33/50

(54) **VERFAHREN UND VORRICHTUNG ZUM MESSEN DER AKTIVITÄT VON ENZYMEN NACH INHIBITORENTZUG**
METHOD AND DEVICE FOR MEASURING THE ACTIVITY OF ENZYMES AFTER INHIBITOR REMOVAL
PROCÉDÉ ET DISPOSITIF DESTINÉS À LA MESURE DE L'ACTIVITÉ D'ENZYMES APRÈS EXTRACTION DE L'INHIBITEUR

(30) Priorität: 08.01.2009 DE 102009004371
(43) Veröffentlichungstag der Anmeldung: 12.10.2011
(73) Patentinhaber: Papst Licensing GmbH & Co. KG, 78112 St. Georgen (DE)
(72) Erfinder: HOFER, Hans Werner, 78464 Konstanz (DE); MEIER, Hans Jörg, 78467 Konstanz (DE)
(74) Vertreter: WSL Patentanwälte Partnerschaft mbB
(86) Internationale Anmeldenummer: PCT/DE2010/000011
(87) Internationale Veröffentlichungsnummer: WO 2010/078868

(56) Entgegenhaltungen:
- WO-A1-97/00969
- WO-A1-2005/070546
- WO-A2-2009/006877
- US-A1- 2003 087 426
- GUO W ET AL: "Crosslinked mercerized cellulose membranes for the affinity chromatography of papain inhibitors" JOURNAL OF MEMBRANE SCIENCE, ELSEVIER SCIENTIFIC PUBL.COMPANY. AMSTERDAM, NL LNKD- DOI:10.1016/S0376-7388(01)00619-6, Bd. 197, Nr. 1-2, 15. März 2002 (2002-03-15), Seiten 53-62, XP004334309 ISSN: 0376-7388

## Beschreibung

Die vorliegende Erfindung betrifft ein Verfahren und eine Vorrichtung zum Messen der Aktivität von Enzymen nach Inhibitorentzug insbesondere bei proteolytischen Enzymen, vor allem bei Cystein-Proteinasen, die für die Diagnose und auch für die Therapie von malignen Tumoren zum Tragen kommen.

In einer Messprobe sind Enzym-Inhibitor-Komplexe, und die proteolytischen Enzyme sind in vivo durch Bindung von Inhibitoren in ihrer Enzymaktivität überwiegend gehemmt. Ein ähnlicher Themenkreis ist aus der WO97/00969 bekannt.

Ferner betrifft die deutsche Patentanmeldung 10 2007 017 681.5 eine ähnliche Erfindung ebenso wie die deutschen Patentanmeldungen 10 2007 057 388.1 und 10 2007 034 120.7, auf deren gesamte Offenbarung Bezug genommen wird.

Aus diesem Stand der Technik ist die Messung der Enzymaktivität solcher Enzyme bekannt, die in der Probe durch Inhibitoren vorwiegend inhibiert sind, und die dadurch zu messen ist, dass die Messprobe zuvor über eine Durchfluss- oder Durchlaufsäule gegeben wird, auf welcher der Probe die Inhibitoren entzogen werden, die das Enzym inhibieren. Danach wird das inhibitorfreie Enzym in eine Messzelle gegeben, in der nach Zugabe eines geeigneten Substrats die Aktivität des Enzyms gemessen wird, z. B. durch den Anstieg der Konzentration mindestens eines Spaltprodukts dieses Substrats über der Zeit.

Es ist Aufgabe der vorliegenden Anmeldung ein Verfahren und eine Vorrichtung zum Messen der Aktivität von Enzymen nach Inhibitorentzug bereitzustellen, die eine erhebliche Genauigkeitssteigerung und größere Zuverlässigkeit der Messungen ergibt, wodurch operative Eingriffe zur Gewinnung der biologischen Probe nicht mehr nötig sind, weil man stattdessen Blutserum als biologische Probe verwenden kann.

Darüberhinaus soll durch die vorliegende Erfindung ein Verfahren und eine Vorrichtung zum Messen der Aktivität von Enzymen nach Inhibitorentzug bereitgestellt werden, die für die Alltagspraxis, z. B. von Arztpraxen bzw. kleinen ärztlichen Labors oder u. U. auch für Notfallsituationen bei relativ einfacher Handhabung ausreichend genaue Messwerte als Prognostikfaktor (für Therapieentscheidungen) oder Marker (für Diagnosezwecke) liefert und insbesondere kostengünstig Ergebnisse liefert.

Diese Aufgabe wird mit dem Verfahren zum Messen der Aktivität von Enzymen nach Inhibitorentzug nach Anspruch 1 und mit der Vorrichtung zum Messen der Aktivität von Enzymen nach Inhibitorentzug nach Anspruch 10 gelöst.

Zusätzliche Weiterbildungen und Ergänzungen ergeben sich aus den Unteransprüchen.

Zur Aktivitätsmessung von Enzymen mittels Inhibitorentzug haben sich fluorogene Substrate als vorteilhaft erwiesen, in denen 7-Amino-4-methylcumarin als Fluorogen an den C-Terminus eines Oligopeptids, vorwiegend eines Dipeptids gebunden ist, dessen N-Terminus mit der Carboxybenzyl-Schutzgruppe (abgekürzt: Cbz bzw. Z) geschützt ist.

Dabei ist jedoch die Eindeutigkeit und damit die Genauigkeit des Messergebnisses für manche Aufgaben noch nicht unbedingt ausreichend.

Durch Verwendung von 7-Amino-4-trifluormethylcumarin als C-Terminus anstelle von 7-Amino-4-methyl-cumarin wird jedoch das Emissionsspektrum des abgespalteten Fluorogens nun weiter in den langwelligen Bereich verschoben, sodass die Fluoreszenz des abgespaltenen Fluorogens in einem Wellenlängenbereich beobachtet werden kann, in dem andere Lumineszenzen aus der zu untersuchenden Lösung das Messergebnis nicht mehr stören, was zur Folge hat, dass man zu höheren Empfindlichkeiten bei der Messung der Aktivität solcher Enzyme vorstoßen kann, insbesondere bei gattungsgemäßen Vorrichtungen und Verfahren.

Im Fall, dass die biologische Probe, z. B. eine Gewebeprobe bzw. ein Gewebehomogenat ist, lässt sich durch den Einsatz eines Bypasses nach Passage der Probe durch den Bypass der Anteil an Enzymaktivität, z. B. der Cysteinprotease, messen, der in dieser Probe ursprünglich nicht inhibiert ist. Es ist bekannt, dass in Gewebehomogenaten nicht die gesamte zu messende Protease inhibiert ist, sondern nur ein Teil. Damit lässt sich aus zwei Aktivitätsmessungen, nämlich nach Passage der biologischen Probe durch die Affinitätschromatographiesäule und durch den Bypass aus der Differenz auch die Aktivität des in der biologischen Probe ursprünglich inhibierten Enzyms bestimmen.

Durch die Erfindung sind Wellenlängenbereiche für die Fluoreszenzemission von ca. 500 nm und mehr möglich.

Die Figuren zeigen:
Fig. 1 eine Vorrichtung zum Messen der Aktivität von Enzymen nach Inhibitorentzug gemäß eines ersten Ausführungsbeispiels der vorliegende Erfindung,
Fig. 2 eine gegenüber der Fig. 1 in der Funktion erweiterte und quasi halbautomatisierbare Vorrichtung, und
Fig. 3 eine Vorrichtung zum Messen der Aktivität von Enzymen nach Inhibitor entzug gemäß eines weiteren Ausführungsbeispiel der vorliegenden Erfindung.

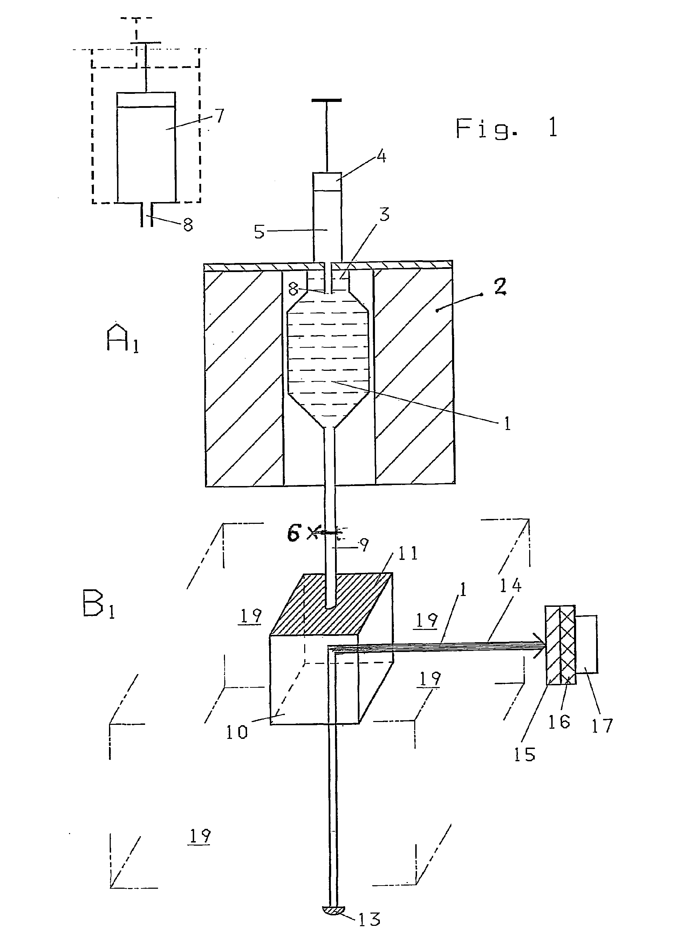

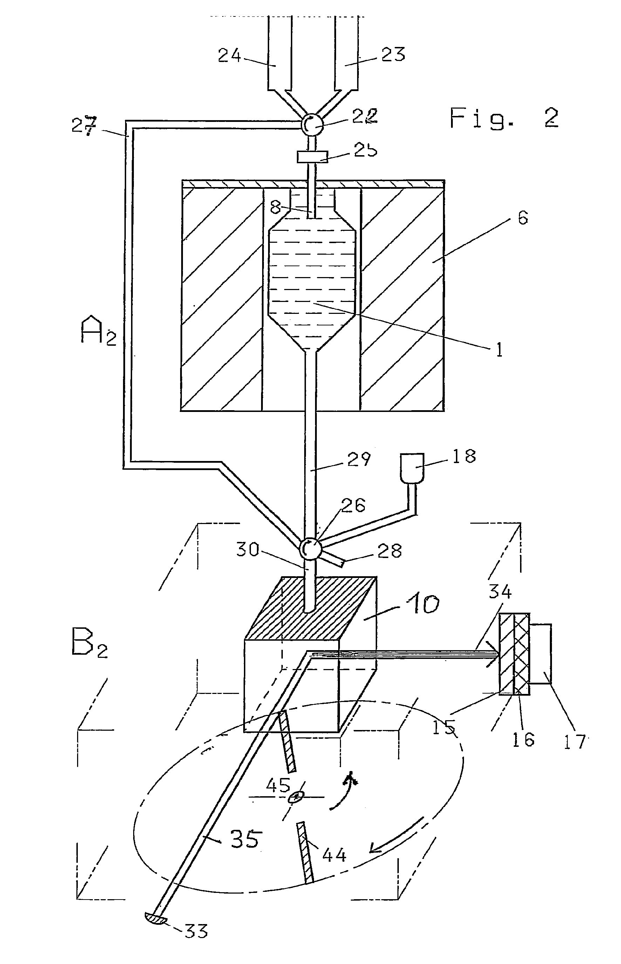

Wobei in den Figuren 1 und 2 jeweils Modul A als ebenes Schnittbild dargestellt, wohingegen Modul B in räumlicher Darstellung gezeigt ist.

In den Figurenbeschreibungen bezeichnen identische Bezugszeichen gleich wirkende oder identische Elemente.

Fig. 1 zeigt eine Vorrichtung zum Messen der Aktivität von Enzymen nach Inhibitorentzug gemäß eines ersten Ausführungsbeispiels der vorliegende Erfindung.

Dabei ist eine austauschbare Affinitäts-Chromatographiesäule 1 von einem Thermostaten 2 umgeben, der mindestens ein Peltierelement aufweist. Die Säule 1 besteht im wesentlichen aus einem räumlichen Zylinder, der mit poröser Substanz, einem Träger, der als Gel fungiert, gefüllt ist, an das eine Substanz gebunden ist, die den Inhibitor des Enzym-Inhibitor-Komplexes stärker bindet als es das Enzym tut, wodurch das Enzym zur Aktivitätsmessung freigesetzt wird. In die obere Öffnung 3 der Säule 1, an deren unterem Ende zweckmäßigerweise ein Sperrventil 6 angebracht ist, ragt die Spitze 8 einer austauschbaren Spritze 4, welche in einem Raum 5 die Messprobe mit dem Enzym-Inhibitor-Komplex enthält.

Das Volumen 5 wird mit ausgedrückter Spritze 4 faktisch zu Null, und der Inhalt entleert sich in die Säule 1, in der sich vorzugsweise ein fest gepackter Träger befindet.

Ein Elutionspuffer vom ca. 100-10³fachen Volumen der Messprobe wird danach mittels einer zweiten Spritze 7 ganz oder teilweise in die Säule 1 eingebracht.

Eine erste Vorgehensweise ist folgende: Man belässt die Messprobe mit einem Teil des Elutionspuffers in der Säule 1 bei wohldefinierter Temperatur (z. B. ca. 4° C) für eine bestimmte Inkubationszeit, in der Praxis ca. 10-18 min. Insbesondere 15 min. haben sich als effektiv erwiesen. Danach wird durch weitere Zugabe von Elutionspuffer mit Hilfe der Spritze 7 das freie Enzym eluiert und die Elutionslösung fließt bei offenem Sperrventil 6 nach unten in Modul B gemäß Fig. 1.

Bei der ersten Methode ist das Ventil 6 zunächst offen, bis der Säulenpuffer in die Säule 1 teilweise eingelaufen ist bzw. bis die Messprobe auf der Säulenlänge verteilt ist. Solange kann auch ein Volumen, das bei der Messung nicht berücksichtigt werden soll, abfließen (z. B. wie in Fig. 2 beschrieben über den Kanal 28). Nach der Inkubationszeit wird zur Elution dieses Ventil 6 geöffnet, dann jedoch wieder geschlossen, damit ein Restvolumen nicht die Messung verschlechtert. Auch dieses Restvolumen kann über den Kanal 28 entsorgt werden.

Eine zweite alternative Maßnahme besteht darin, dass die Messprobe mit Hilfe des zugegebenen Säulenpuffers von oben nach unten durch die Säule wandert mit einer Geschwindigkeit die gewährleistet, dass auf diesem Weg der Inhibitor von allen in der Messprobe enthaltenen Enzym-Inhibitorkomplexen auf die auf der Säule immobilisierte Substanz übergeht, die den Inhibitor stärker bindet als es das Enzym tut. Das ist quasi eine Wanderungsinkubation. Dadurch wird das freie Enzym eluiert und die Elutionslösung fließt nach unten in das Modul B gemäß Fig. 1.

Auch hier wird ein Verwerfungsvolumen anfangs (wie in Fig. 2 mittels Kanal 28 beschrieben) entsorgt; ebenso geschieht dies nach dem Durchlauf des quasi optimalen Messvolumens.

Das Modul A in Fig. 1 stellt also eine Vorrichtung für den Inhibitorentzug dar, die sich im wesentlichen räumlich über der Messbox Modul B befindet, wobei der Durchlauf von der Säule 1 in das Modul B gemäß der Fig. 1 mittels Rohr- oder Schlauchleitung durch einen Deckel 11 hindurch in eine Fluoreszenzküvette 10 zustande kommt. (Der Deckel ist geschwärzt zur Absorption des durch die Messprobe gelangten Laserlichts). Das freie Enzym spaltet gemäß Enzymassay als proteolytisches Enzym aus dem in die Küvette 10 gegebenen Substrat ein Fragment ab, das in freier Form fluoresziert. Aus dem zeitlichen Anstieg dieser Fluoreszenzintensität lässt sich die Enzymaktivität des freigesetzten Enzyms bei definierter Temperatur (welche mit Hilfe von Peltierelementen 19 eingestellt wird) bestimmen. In der unteren Messbox (Modul B) befindet sich eine Laserdiode 13 zur Anregung dieser Fluoreszenz. Die Laserdiode emittiert zweckmäßigerweise Licht der Wellenlänge, die dem Excitationsmaximum des fluoreszierenden Substratfragments entspricht. Das emittierte Fluoreszenzlicht 14 wird senkrecht zur Einstrahlrichtung mit Hilfe einer Fotodiode 17 detektiert. Kantenfilter 15 und Interferenzfilter 16 filtern nahezu alles Streulicht, das vom Anregungslicht stammt, aus und lassen nur Fluoreszenzlicht auf die Fotodiode 17 fallen.

Die Temperierung der Affinitätschromatographiesäule 1 im Modul A erfolgt bei 3-20° C, vorzugsweise bei 4-5° C, da mit der Bindung des Inhibitors an das Affinitäts-Chromatographiesäulen-Material das proteolytische Enzym freigesetzt wird, das sich selbst verdauen kann, d. h. bei höheren Temperaturen greift ein proteolytisches Enzymmolekül das andere an.

Die Messung der Enzymaktivität in Modul B erfolgt temperiert vorzugsweise bei 37° C (für humanmedizinische Zwecke), (wozu eine Zusatzeinrichtung für die Konstanthaltung der Temperatur zweckmäßigerweise wiederum mittels Peltierelementen dienlich ist: Thermostat 19). Sie kann aber auch beispielsweise bei 20 °C bzw. bei Raumtemperatur gemessen werden, jede gewählte Temperatur muss aber über die gesamte Messzeit konstant gehalten werden.

Im einfachsten Fall ist auch die Küvette 10 als Wegwerfartikel ausgestaltet. (Sie kann vorher gefüllt sein mit Messpuffer und den Ingredientien gemäß Enzymassay). Jedoch kann auch eine Zufuhr dieser Mischung direkt in die Küvette 10 erfolgen, oder u. U. im Kanal 9 mittels zusätzlichem Ventil und Pumpe, je nach Bedarf auch noch mit einem Messpuffer geeigneter Art versetzt. Nach Abschluss der Elution wird die enzymatische Reaktion durch Zugabe von Substrat gestartet. Diese kann z. B., wie in Fig. 2 dargestellt, aus einem Substratbehälter 18 über das Ventil 26 erfolgen, oder wie gemäß Fig. 1, über eine nicht dargestellte Spritze durch den Abschlussdeckel 11 hindurch eingebracht werden.

Die Teile 1, 5, 4, 10 können als (billige) Wegwerfartikel ausgestaltet sein. Der Vorteil der austauschbaren Teile liegt darin, dass keinerlei Bestandteile einer Probe eines Patienten mit einer anderen Probe eines anderen Patienten zusammenkommen können! Man muss sich vor Augen halten, dass die Körperflüssigkeit des Patienten als Messprobe auf die Säule 1 gegeben wird und vor allem nur der Enzym-Inhibitor bei der Elution auf der Säule bleibt; es ist aber nicht klar, ob auch andere Bestandteile auf der Säule noch zurückbleiben. Auf jeden Fall enthält die Messprobe in der Fluoreszenzküvette 10 nicht nur das freie Enzym, sondern auch den größten Teil der anderen Bestandteile der ursprünglichen Körperflüssigkeit. Ein weiterer Vorteil dieses Konzepts besteht darin, dass komplizierte mechanische Teile wie Ventile/ Pumpen entfallen.

Die Verdünnung des Eluats mit Messpuffer kann für ein gutes Messergebnis vorteilhaft, evtl. notwendig sein. Die Kombination einer Laserdiode und/oder Fotodiode direkt an der Messküvette führt zu einer hohen Nachweisgrenze.

In Fig. 2 ist eine gegenüber der Fig. 1 in der Funktion erweiterte und quasi halbautomatisierbare Vorrichtung dargestellt, jedoch kann auch die Säule 1 wie oben für Fig. 1 mit den zwei Methoden betrieben werden. Daher sind vor und nach der Säule 1 Mehrweg-Ventile 22, 26 vorgesehen. Teile derselben können auch mit einer Anordnung der Fig. 1 kombiniert werden.

In Fig. 2 ist ebenfalls eine Affinitätschromatographiesäule 1, welche von einer temperierbaren Einheit 6 (z. B. Peltierelement) umgeben vorzugsweise auf 4° C eingestellt ist. Im Inneren der Säule ist fest gepacktes Material, an welche eine Substanz gebunden ist, die eine höhere Affinität zu den Inhibitoren aufweist als ein hier interessierender Enzyminhibitorkomplex der Messprobe, z. B. ist an das Sepharosegel als Packungsmaterial Papain als Substanz gebunden, und die Messprobe enthält z. B. den Enzym-Inhibitorkomplex von Cathepsin B. Nach Einlass der Messprobe aus einem Behälter über einen Tubus 23, welcher über ein Ventil 22 (durch einen Drehpfeil gekennzeichnet) geschieht, wird aus dem Zufluss 24, wobei das Ventil 22 umgestellt wird, ein Säulenpuffer in die Säule 1 gelassen.

Der Tubus 23 kann ein Wegwerfteil sein oder/und als Zufluss aus einem Behälter dienen, der für weitere Messungen benutzt werden kann. Der Kanal 24 kann als eine weitere austauschbare 2. Spritze als Wegwerfteil aus Kunststoff mit einer bestimmten Dosis Volumen, die im allgemeinen das Vielfache vom Volumen der Messprobe beträgt, ausgestattet sein, oder er kann einfach ein Säulenpufferreservoir sein oder zu einem solchen führen, wobei bei der entsprechenden Stellung des Ventils 22 in den Eingang der Säule 1 der Durchfluss für den Säulenpuffer freigegeben wird.

Die Pumpe 25 ist nach dem Ventil 22 angeordnet, um bei Bedarf einen beliebigen Druck (also auch etwa Nullwert) zum optimalen Durchlauf durch die Säule zu erzeugen. Das Ventil 22 kann auch so gestellt sein, dass die Messprobe und/oder der Säulenpuffer eine Bypass-Leitung 27 zum unteren Ausgang der Durchflusssäule 1 oder zum Ventil 26 führt. Am Ausgang der Säule 1 ist dieses weitere Ventil 26 vorgesehen (wiederum mit einem Drehpfeil angedeutet), und zwar auch für folgenden Zweck: Wenn die Messprobe durch die Säule 1 läuft, wird ein erster Teil des Volumens verworfen und im Ausgang 28 entsorgt. Sodann wird das Drehventil umgestellt auf Durchflussrichtung 30 zur Messbox B hin, denn dieses weitere Volumen ist dann besser geeignet für eine genaue Messung. Der Rest der Elutionsflüssigkeit wird dann wieder zum Ausgang 28 hin abgeleitet.

Für die Ermittlung des Verwerfungsvolumens wird die Verdünnung der Probe nach dem Durchlauf durch die Säule 1 ermittelt. Eventuell muss man eine einfache Vorrichtung vorsehen zur Bestimmung des Volumens, das verworfen und über den Kanal 28 abgeleitet wird, daraus lässt sich dann das Volumen der eluierten Messprobe mit dem freien Enzym bestimmen als Differenz zu dem über 24 aufgetragenen Elutionspuffer.

Nach Durchfluss durch den Kanal 30 in einen (z. B. kubusförmigen oder quaderförmigen) Behälter 10 läuft die Messprobe, deren Enzym von Inhibitoren befreit ist, in diesen Behälter 10, welcher zuvor mit Messpuffer und Ingredientien gemäß Enzymassay versehen wurde. Zum Start der Enzym-Reaktion wird die optimale Dosis von Substrat zugegeben aus Vorratsbehälter 18.

Der Behälter 10 kann ebenfalls für einfachste Bedürfnisse als Wegwerfartikel ausgestaltet sein, indem der Kubus 10 z. B. aus Kunststoff besteht.

Er kann eine Fluoreszenzküvette sein, wobei im Modul B eine Laserdiode 33 vorgesehen ist, die eine Wellenlänge λ von etwa 400 nm aufweist.

Diese Laserstrahlung aus 33 ist etwa senkrecht zur Fallrichtung, d. h. etwa 90° zur Einflussrichtung vom Kanal 29 oder 30. Senkrecht dazu (vom Betrachter aus nach rechts im Winkel von 90°) ist als Reaktion des Spaltprodukts eine Fluoreszenzstrahlung 34 dargestellt, die auf einen Kantenfilter 15, parallel dazu einen Interferenzfilter 16, und weiter parallel dazu auf eine Fotodiode 17 trifft, die an ihrem Ausgang die Intensität der Fluoreszenzstrahlung zu messen gestattet (wie in Fig. 1). Die Strahlungen 34, 35 liegen also in einer Ebene, die vorzugsweise senkrecht zur Fallrichtung ist. Dies entspricht im wesentlichen einer Detektion der Fluoreszenzemission nach dem off-axis-Verfahren.

Unterhalb der Fluoreszenzküvette 10 ist ein Magnetrührgerät 44 um die Achse 45 rotierend angedeutet, das die Mischung möglichst homogen machen soll. Fluoreszenz beginnt sofort, nachdem die Partikel der Messprobe auf Substratteile treffen, die Intensität ist proportional der Konzentration des Spaltprodukts und dieses wiederum ist ein proportionales Maß für die Enzymaktivität.

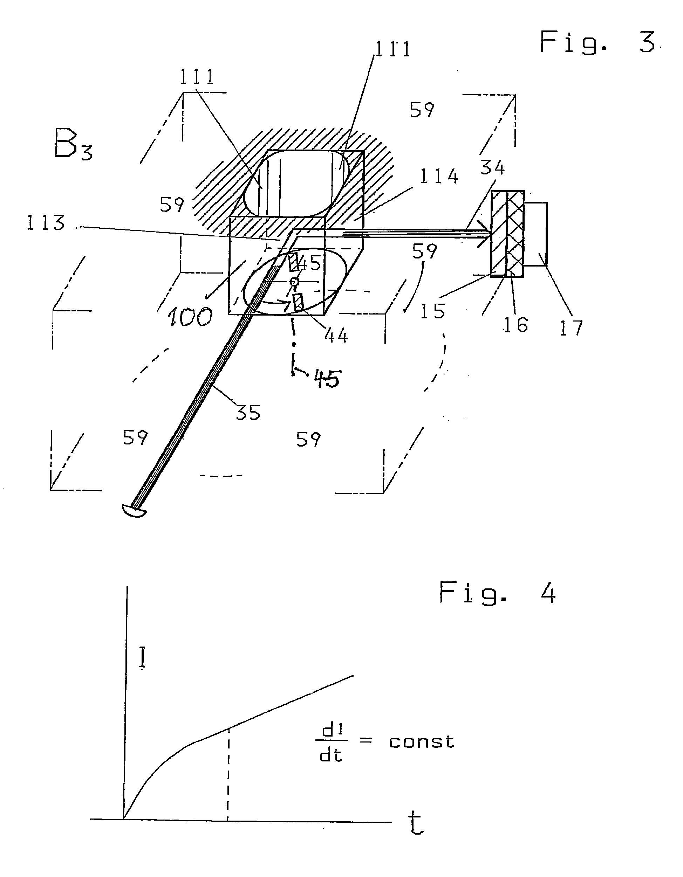

Die Messkurve für die emittierte Fluoreszenzintensität in Fig. 4 geht nach einer Anfangsphase in eine Gerade über, und sobald der Kurvenverlauf geradlinig ist, wird deren Anstieg festgestellt und man hat das gewünschte Ergebnis dl/dt. Die Fig. 4 ist für alle anderen Figuren repräsentativ.

Im einfachsten Fall ist das Modul A eine Wegwerf-Chromatographiesäule, in welche oben eine erste Einmalspritze mit der Messprobe eingeführt wird, danach eine 2. Spritze mit dem Säulenpuffer, der Ausgang 9 kann unmittelbar über das Ventil 6 mit der Messbox 10 verbunden werden, wobei diese dann auch eine (wie beschrieben) aufbereitete, billige Wegwerfpackung ist. Das Modul B, auf jeden Fall die Messbox 10, sollte temperiert werden, wozu um die Messbox 10 herum ein weiteres Peltierelement 16 in unmittelbarer Umgebung vorgesehen ist. (Das kann für humanmedizinische Anwendung vorzugsweise auf 37° C eingestellt sein). Alternativ kann also ein Substratbehälter 18 vorgesehen sein, der einen direkten Kanal zur Box 10 hin aufweist. Vorzugsweise erfolgt die Zufuhr zur Box 10 jedoch aus dem Substratbehälter indirekt über den Kanal 9 bzw. 29 bzw. 30.

Im einfachsten Fall ist Modul B eine mit Messpuffer und weiteren Ingredientien, wie z. B. ein nicht-ionisches Tensid und Dithiothreitol oder Cystein, aufbereitete kostengünstig Wegwerfpackung.

Das Ventil ist dann unerlässlich, wenn man die Messprobe im Durchflussverfahren aus der Säule eluiert, denn dann wird man einen ersten Teil des Eluats verwerfen. Es ist aber auch dann unerlässlich, wenn man die Probe auf der Säule eine Zeit lang inkubiert; denn dazu muss man nach dem Auftragen der Probe auf die Säule danach einen gewissen Teil des Säulenpuffers in die Säule lassen und unten auslassen; dadurch sickert die Messprobe in die Säule ein und die Enzym-Inhibitorkomplexe der Messprobe finden so möglichst alle Kontakt mit der auf der Säule immobilisierten Substanz, die den Inhibitor besser bindet.

Während in der Anordnung gemäß Fig. 1 die Laserstrahlung 14 und das emittierte und detektierte Fluoreszenzlicht parallel zur oder in der Zeichnungsebene bzw. Schnittebene von Modul A verlaufen, liegen diese Strahlungen 34, 35 in Fig. 2 in einer Ebene senkrecht zur Schnittebene von Modul A, d. h. im allgemeinen senkrecht zur Fallrichtung oder Durchflussrichtung in Modul A (Detektion der Fluoreszenzemission nach dem off-axis-Verfahren).

Dadurch ist unterhalb der Messküvette 10 bzw. des Moduls B ein Mischer zur Homogenisierung für den Inhalt der Küvette 10 räumlich geschickt und für einfache Handhabung vorteilhaft angeordnet, z. B. als magnetisches Rührwerk 44, 45.

Eine weitere Ausführungsvariante gemäß der vorliegenden Erfindung ergibt sich folgendermaßen: Eine poröse Substanz, der Träger, an den die inhibitorbindende Substanz gebunden ist, die den Inhibitor des Enzym-Inhibitor-Komplexes stärker bindet als das Enzym, kann überraschenderweise auch durch einen Zellulosestreifen ersetzt werden, an den die Inhibitorbindesubstanz adsorptiv oder kovalent, vorzugsweise kovalent gebunden ist. Bei der kovalenten Anbindung hat es sich besonders vorteilhaft erwiesen, den letzten Schritt dieser Anbindung insbesondere photochemisch auszuführen.

Eine kovalente Anbindung von Papain an Cellulose geschieht in drei Stufen:
1. Aktivierung der freien Hydroxylgruppen von Cellulose, z. B. mit Perjodat.
2. Kopplung eines bifunktionellen Reagens an die aktivierte Cellulose.
3. Reaktion mit Papain.

Experimentell wurde im 1. Schritt ein Rundfilter mit einem Durchmesser von 110 mm mit Perjodat behandelt. Der zweite Reaktionsschritt wurde mit dem heterobifunktionellen Reagens p-Azidobenzoylhydrazid (ABH) vollzogen. Die Phenylazidogruppe eignet sich nach Photoaktivierung zur kovalenten Kopplung an CH- und NH-Gruppen von Proteinen, welche durch Wasserstoffbrücken aktiviert sind. Damit wurde im dritten Schritt der ABH-behandelte Rundfilter auf eine langweilige (310 nm) UV-Lichtquelle gelegt und vor der Belichtung mit Papain-Lösung überschichtet, diese Prozedur wurde auf beiden Seiten des Filters ausgeführt.

Die Kopplungseffizienz von Papain an Cellulose wurde mit Hilfe eines fluorometrischen Aktivitätstests und Arg-Arg-AMC als Substrat mit der Papain-Aktivität an Papain-Agarose (250 µg/ml Gel) verglichen.
Hierzu wurde der Filter in 32 Segmente ungefähr gleicher Größe aufgeteilt. Ein Segment hat eine Fläche von ca. 3 cm².

Dabei wurden folgende Mittelwerte gemessen (n=3):

| **Material** | **mg Träger** | **rel. Fluoreszenz (netto)** | **µg Papain** |
|---|---|---|---|
| Papain-Cellulose | 18 | 1454 | 16,9 |
| Papain-Agarose | 1,2 | 1076 | 12,5 |

Die Papiersegmente und die für den Cathepsin-B-Test verwendete Agarose enthielten also vergleichbare Mengen an Papain.

Drei verschiedene Seren wurden bezüglich der messbaren Protease-Aktivitäten mit dem Substrat Z-Arg-Arg-AFC vor und nach Behandlung mit Papain, welches entweder auf Cellulose-Filterpapier oder auf Agarose immobilisiert war, miteinander verglichen. Dafür wurden entweder 50 µl Papain-Agarose oder ein 1/32 Segment der wie oben beschriebenen Papain-Cellulose eingesetzt.

| | **Serum 1** | **Serum 2** | **Serum 3** |
|---|---|---|---|
| Relative Fluoreszenz (unbehandelt) | 1835 ± 249 | 1805 ± 50 | 1791 ± 53 |
| rel. Fluoreszenz (mit Papain-Cellulose behandelt) | 3806 | 4702 | 4757 |
| rel. Fluoreszenz (mit Papain-Agarose behandelt) | 3732 | 4995 | 4478 |
| Aktivität Papain-Cellulose behandelt | 0,44 | 0,65 | 0,66 |
| Aktivität Papain-Agarose behandelt | 0,42 | 0,71 | 0,60 |

Aktivitäten sind in nmol Substratumsatz pro Minute und ml Serum angegeben. Die Inkubationsdauer betrug 60 min. bei 37 °C.

Bei der Deinhibierung der biologischen Probe mit einem solchermaßen präparierten Zellulosestreifen entfällt die gesamte Vorrichtung gemäß Modul A (Fig. 1).

Stattdessen wird in einer einfachen Variante zur Deinhibierung der präparierte Zellulosestreifen in die mit Messpuffer bereits verdünnte biologische Probe getaucht, die sich hierfür in einem temperierbaren Reaktionsgefäß befindet. Die Messprobe wird hierzu temperiert, vorzugsweise auf 20 °C, es ist aber auch jede andere Temperatur insbesondere zwischen 4 °C und 20 °C denkbar. Nach erfolgter Deinhibierung, die bei beispielsweise 20 °C nach 5 min. abgeschlossen ist, wird der Zellulosestreifen aus der Messprobe entfernt und anschließend die Enzymaktivitätsmessung durchgeführt. Wenn hierzu die Endpunktbestimmung gewählt wird, wird ein Teil der deinhibierten Messprobe in ein weiteres temperierbares Reaktionsgefäß gebracht, in dem sich bereits die für den Enzymassay notwendigen Ingredienzen und das Substrat befinden.

Bei geschlossenem Reaktionsgefäß wird die Probe bei der gewählten Messtemperatur gehalten. Nach einer vorbestimmten Zeit, vorzugsweise 30 bis 40 min., wird das Reaktionsgefäß mit Deckel geschüttelt, um das am Deckel kondensierte Wasser wieder in die Probe zurückzuführen. Dann wird die Probe in den Messtopf 111 überführt und die Fluoreszenzintensität gemesssen, aus der mit Hilfe einer Eichkurve auf die Aktivität des in der Messprobe vorhandenen deinhibierten Enzyms geschlossen werden kann.

Die Fluoreszenzemission kann wie in Fig. 3 angedeutet im Off-Axis-Verfahren gemessen werden oder mit Hilfe eines kleinen, handlichen, handelsüblichen Fluorimeters, der nach dem Prinzip des Auflichtverfahrens funktioniert oder in einer Mikrotiterplatte mit einem Fluoreszenzreader, der ebenfalls nach dem Prinzip des Auflichtverfahrens funktioniert.

Ist die Messprobe ein Gewebehomogenat, so lässt sich durch Messung der Enzymaktivität ohne Deinhibierung der Anteil der Enzymaktivität messen, die nicht inhibiert ist, und aus dem Enzymaktivitätswert nach Deinhibierung mit dem präparierten Cellulosestreifen erhält man die Aktivität des in der biologischen Probe insgesamt vorhandenen Enzyms und aus der Differenz beider Messwerte erhält man den Anteil des Enzyms, der in der biologischen Probe inhibiert ist.

Alternativ zu einem Zellulosestreifen kann man auch einen Stift aus porösem Material als festes Trägermaterial verwenden, beispielsweise poröse Keramik mit einem genügend großen Hohlvolumen, an der die Inhibitorbindesubstanz adsorptiv oder kovalent, vorzugsweise kovalent gebunden ist. Dabei weist das Trägermaterial eine ausreichende Eigenstabilität sowie ein maximal großes Hohlvolumen mit entsprechend großflächiger Gesamtinnenwand auf.

In der obigen Beschreibung wird für die humanmedizinische Anwendung vorzugsweise eine Messtemperatur für die Enzymaktivitätsmessung von 37 °C angegeben. Das ist jedoch nicht als eine strikte Anweisung zu verstehen. Versuche zwischen 20 °C und 40 °C Messtemperatur ergaben Messzeiten zwischen 120 und 20 Minuten.

Die vorliegende Erfindung wurde im vorangehenden anhand bevvorzugter Ausführungsbeispiele beschrieben, ist jedoch darauf nicht beschränkt, sondern ergibt sich insbesondere aus den nachfolgenden Ansprüchen.

## Patentansprüche

1. Verfahren zur Bestimmung der Aktivität von Enzymen in einer Messprobe, die wenigstens ein Enzym und wenigstens einen zu dem Enzym korrespondierenden Enzyminhibitor enthält, indem nach Entzug des Inhibitors das befreite Enzym in seiner Aktivität dadurch gemessen wird, dass der Messprobe ein Substrat zugeführt wird, und der zeitliche Verlauf der Konzentration zumindest eines Reaktionsprodukts, insbesondere eines Spaltprodukts erfasst wird und das enzymspezifische Substrat ein Fluorogen trägt, welches bei der enzymatischen Reaktion abgespalten wird und dessen Fluoreszenz als Messparameter in einem Wellenlängenbereich detektiert werden kann, wo der Messparameter eindeutig der zu messenden Enzymaktivität zugeordnet werden kann,
**dadurch gekennzeichnet, dass**
der Entzug des Inhibitors durch Eintauchen eines festen Trägers, an welchem die Inhibitorbindesubstanz fixiert ist, in die Messprobe erfolgt.

2. Verfahren nach Anspruch 1, **dadurch gekennzeichnet, dass** das Trägermaterial Cellulose ist, an welches die Inhibitorbindesubstanz kovalent gebunden ist.

3. Verfahren nach Anspruch 2, **dadurch gekennzeichnet, dass** die Anbindung der Inhibitionssubstanz an Cellulose chemisch oder photochemisch erfolgt.

4. Verfahren nach Anspruch 2 oder 3, **dadurch gekennzeichnet, dass** die Deinhibierung bei einer Temperatur zwischen 4 °C und 30 °C, vorzugsweise bei 20 °C, dadurch erfolgt, dass der Cellulosestreifen mit der kovalent gebundenen Inhibitorbindesubstanz in die Messprobe eingetaucht und nach erfolgter Deinhibierung wieder herausgezogen wird, und anschließend ein Teil dieser deinhibierten Messprobe der Enzymaktivitätsmessung zugeführt wird.

5. Verfahren nach einem der vorausgehenden Ansprüche, **dadurch gekennzeichnet, dass** die Deinhibierung in einem ersten temperierbaren Reaktionsgefäß bei konstanter Temperatur erfolgt, die enzymatische Reaktion mit dem fluorogenen Substrat in einem zweiten temperierbaren Reaktionsgefäß, in den ein Teil der deinhibierter Probe transferiert wird, bei konstanter Temperatur erfolgt, und in einem Messtopf danach die Fluoreszenzintensität gemessen wird.

6. Verfahren nach einem der vorausgehenden Ansprüche, **dadurch gekennzeichnet, dass** die Fluoreszenzemission nach dem off-axis-Verfahren oder nach dem Auflichtverfahren gemessen wird.

7. Verfahren nach einem der vorausgehenden Ansprüche, **dadurch gekennzeichnet, dass** die Enzyme Proteasen sind und das fluorogene Substrat ein Oligopeptid ist, dessen N-Terminus mit einer Schutzgruppe geschützt ist.

8. Verfahren nach einem der vorausgehenden Ansprüche, **dadurch gekennzeichnet, dass** als Schutzgruppe für den N-Terminus die Carboxybenzylgruppe verwendet wird.

9. Verfahren nach einem der vorausgehenden Ansprüche, **dadurch gekennzeichnet, dass** als Oligopeptid ein Dipeptid verwendet wird, an dessen C-Terminus 7-Amino-4-trifluormethylcumarin gebunden ist.

10. Vorrichtung zur Durchführung des Verfahrens nach einem der vorausgehenden Ansprüche 1 bis 9, mit:
- einem ersten temperierbaren Reaktionsgefäß zur Deinhibierung,
- einem zweiten temperierbaren Reaktionsgefäß, welches verschließbar ist, zur Durchführung einer enzymatischen Reaktion in einem Teil der deinhibierten Messprobe, und
- einem dritten Reaktionsgefäß, welches als Messtopf dient, und in welchem die Fluoreszenzemission bestimmt wird, und
- einem festen Träger, an welchem die Inhibitorbindesubstanz fixiert ist und der für das Eintauchen in die Messprobe ausgelegt ist.

11. Vorrichtung nach Anspruch 10, **dadurch gekennzeichnet, dass** der feste Träger von stiftartiger Gestalt ist und Teil eines vorzugsweise automatisch betriebenen Zuführapparates oder eines Handgerätes ist.

12. Vorrichtung nach Anspruch 11, **dadurch gekennzeichnet, dass** der Zuführapparat Teil eines Fluoreszenzreaders ist.

13. Vorrichtung nach einem der vorausgehenden Ansprüche 11 oder 12, **dadurch gekennzeichnet, dass** das Handgerät in Verbindung mit einem Fluorimeter arbeitet.

14. Vorrichtung nach einem der vorausgehenden Ansprüche 10 bis 13, **dadurch gekennzeichnet, dass** der feste Träger ein Stift aus porösem Material als Trägermaterial ist, vorzugsweise aus poröser Keramik.

## Claims

1. Method for measuring the activity of enzymes in a sample which contains at least one enzyme and at least one enzyme inhibitor corresponding to said enzyme, whereby after removing the inhibitor the activity of the freed enzyme is measured in such a way that a substrate is added to the sample and the time course of the concentration of at least one reaction product, in particular of a cleavage product is recorded and the enzyme specific substrate has a fluorogenic part which is cleaved in the enzymatic reaction and the fluorescence of which can be detected as a measuring parameter in a wavelength range where the measuring parameter can be assigned unambiguously to the enzyme activity to be measured,
**characterized in that**
the removing of the inhibitor is carried out by immersing into the sample a rigid carrier to which the inhibitor binding substance is bound.

2. Method according to claim 1,
**characterized in that**
the carrier is cellulose to which the inhibitor binding substance is covalently bound.

3. Method according to claim 2,
**characterised in that**
the binding of the inhibition substance to cellulose is carried out in a chemical or photochemical way.

4. Method according to claim 2 or 3,
**characterised in that**
the de-inhibition is carried out between 4 °C and 30 °C, preferably at 20 °C, in such a way that the cellulose strip to which the inhibitor binding substance is covalently bound is immersed into the sample and after the de-inhibition is withdrawn from the sample, and afterwards a portion of this de-inhibited sample is provided to the enzyme activity measurement.

5. Method according to one of the preceding claims,
**characterised in that**
the de-inhibition is carried out in a first temperature-controllable reaction vessel at a constant temperature, the enzymatic reaction with the fluorogenic substrate is carried out at a constant temperature in a second temperature-controlled reaction vessel to which a portion of the de-inhibited sample is transferred, and afterwards the fluorescence intensity is measured in a measuring vessel.

6. Method according to one of the preceding claims,
**characterised in that**
the fluorescence emission is measured according to the off-axis method or to the epifluorescence method.

7. Method according to one of the preceding claims
**characterised in that**
the enzymes are proteases and the fluorogenic substrate is a oligopeptide the N-terminus of which is protected by a protecting group.

8. Method according to one of the preceding claims
**characterised in that**
the protecting group of the N-terminus is the carboxybenzylgroup.

9. Method according to one of the preceding claims,
**characterised in that**
the oligopeptide is a dipeptide to the C terminus of which 7-Amino-4-trifluoromethylcoumarin is bound.

10. Device for carrying out the method according to one of the preceding claims 1-9, having:
- a first temperature-controllable reaction vessel for the de-inhibition,
- a second temperature-controllable reaction vessel, which can be closed, for carrying out the enzymatic reaction in a portion of the de-inhibited sample, and
- a third reaction vessel which serves as a measuring vessel and in which the fluorescence emission is measured, and
- a rigid carrier to which the inhibitor binding substance is bound and which is designed so as to immerse it into the sample.

11. Device according to claim 10,
**characterised in that**
the rigid carrier is pin-shaped and part of a preferably automatically driven feeding system or of a hand set.

12. Device according to claim 11,
**characterised in that**
the feeding system is part of a fluorescence reader.

13. Device according to one of the preceding claims 11 or 12,
**characterised in that**
the hand set works in combination with a fluorimeter.

14. Device according to one of the preceding claims 10 to 13,
**characterised in that**
the rigid carrier is shaped as a pin made of porous material, preferably of ceramic which serves as carrier material.

## Revendications

1. Procédé de détermination de l'activité d'enzymes dans un échantillon de mesure, qui contient au moins une enzyme et au moins un inhibiteur d'enzyme correspondant à l'enzyme, dans lequel, après enlèvement de l'inhibiteur, on mesure l'activité de l'enzyme libérée en amenant l'échantillon de mesure à un substrat et on détermine l'évolution dans le temps de la concentration d'au moins un produit de réaction, en particulier d'un produit de dissociation ; le substrat spécifique d'enzyme portant un fluorogène qui est dissocié lors de la réaction enzymatique et dont la fluorescence peut être détectée en tant que paramètre de mesure dans une gamme de longueurs d'onde dans laquelle le paramètre de mesure peut être sans ambiguïté affecté à l'activité enzymatique à mesurer, **caractérisé en ce que** l'enlèvement de l'inhibiteur s'effectue par immersion d'un support solide sur lequel est fixée la substance de liaison à l'inhibiteur.

2. Procédé selon la revendication 1, **caractérisé en ce que** le matériau dudit support est de la cellulose, à laquelle la substance de liaison à l'inhibiteur est liée par liaison covalente.

3. Procédé selon la revendication 2, **caractérisé en ce que** la liaison de la substance d'inhibition à la cellulose a lieu par voie chimique ou photochimique.

4. Procédé selon la revendication 2 ou 3, **caractérisé en ce que** la désinhibition est réalisée à une température comprise entre 4°C et 30°C, de préférence à 20°C, en immergeant la bande de cellulose, portant la substance de liaison à l'inhibiteur liée par liaison covalente, dans l'échantillon de mesure et en l'extrayant à nouveau après que la désinhibition a eu lieu, une partie de cet échantillon de mesure désinhibé étant ensuite envoyée à la mesure de l'activité enzymatique.

5. Procédé selon l'une des revendications précédentes, **caractérisé en ce que** la désinhibition a lieu dans un premier réacteur pouvant être porté à l'équilibre de température, à température constante, la réaction enzymatique avec le substrat fluorogène étant mise en oeuvre à température constante dans un deuxième réacteur pouvant être porté à l'équilibre de température, dans lequel une partie de l'échantillon désinhibé est transférée, l'intensité de fluorescence étant ensuite mesurée dans un creuset de mesure.

6. Procédé selon l'une des revendications précédentes, **caractérisé en ce que** l'émission de fluorescence est mesurée par le procédé off-axis (en-dehors de l'axe) ou par le procédé en lumière réfléchie.

7. Procédé selon l'une des revendications précédentes, **caractérisé en ce que** les enzymes sont des protéases et le substrat fluorogène est un oligopeptide dont le site N-terminal est protégé par un groupe protecteur.

8. Procédé selon l'une des revendications précédentes, **caractérisé en ce qu'**on utilise, en tant que groupe protecteur pour le site N-terminal, le groupe carboxybenzyle.

9. Procédé selon l'une des revendications précédentes, **caractérisé en ce qu'**on utilise, en tant qu'oligopeptide, un dipeptide au site C-terminal duquel est liée une 7-amino-4-trifluorométhylcoumarine.

10. Dispositif pour la mise en oeuvre du procédé selon l'une des revendications 1 à 9, comprenant :
- un premier réacteur pouvant être porté à l'équilibre de température, pour la désinhibition,
- un deuxième réacteur, pouvant être porté à l'équilibre de température, qui peut être fermé, pour la mise en oeuvre d'une réaction enzymatique dans une partie de l'échantillon de mesure désinhibé, et
- un troisième réacteur, qui sert de creuset de mesure, et dans lequel est déterminée l'émission de fluorescence, et
- un support solide, auquel est fixée la substance de liaison à l'inhibiteur, et qui est conçu pour être immergé dans l'échantillon de mesure.

11. Dispositif selon la revendication 10, **caractérisé en ce que** le support solide a une structure en forme de broche, et est une partie d'un appareil de transfert de préférence à entraînement automatique, ou d'un appareil de manutention.

12. Dispositif selon la revendication 11, **caractérisé en ce que** l'appareil de transfert est une partie d'un lecteur de fluorescence.

13. Dispositif selon l'une des revendications précédentes 11 ou 12, **caractérisé en ce que** l'appareil de manutention fonctionne en liaison avec un fluorimètre.

14. Dispositif selon l'une des revendications précédentes 10 à 13, **caractérisé en ce que** le support solide est une broche en un matériau poreux servant de matériau support, de préférence en céramique poreuse.
